# EUROPEAN PATENT SPECIFICATION

(11) **EP 4 066 752 B1**
(45) Date of publication and mention of the grant of the patent: **30.04.2025**
(21) Application number: 22165292.8
(22) Date of filing: 30.03.2022
(51) Int. Cl.: A61B 17/12

(54) **MEDICAL DEVICE DELIVERY MEMBER WITH FLEXIBLE STRETCH RESISTANT DISTAL PORTION**
ABGABEELEMENT EINER MEDIZINISCHEN VORRICHTUNG MIT FLEXIBLEM, DEHNBESTÄNDIGEM DISTALEM ABSCHNITT
ÉLÉMENT DE FOURNITURE DE DISPOSITIF MÉDICAL AVEC UNE PARTIE DISTALE FLEXIBLE RÉSISTANT À L'ÉTIREMENT

(30) Priority: 31.03.2021 US 202117218801; 19.07.2021 US 202117379276
(43) Date of publication of application: 05.10.2022
(73) Proprietor: DePuy Synthes Products, Inc., Raynham, MA 02767 (US)
(72) Inventor: LORENZO, Juan, Raynham, 02767 (US); MONTIDORO, Tyson, Raynham, 02767 (US); SOLAUN, Daniel, Raynham, 02767 (US); BLUMENSTYK, David, Raynham, 02767 (US)
(74) Representative: Carpmaels & Ransford LLP

(56) References cited:
- EP-A1- 1 188 414
- EP-A2- 3 760 139

## Description

### CROSS REFERENCE TO RELATED APPLICATIONS

This application is a continuation in part of U.S. Patent Application No. 17/218,801 filed on March 31, 2021, which is a continuation in part of U.S. Patent Application No. 16/502,767 filed on July 3, 2019, and this application is also a continuation in part of U.S. Patent Application No. 16/592,320 filed on October 3, 2019.

### FIELD OF INVENTION

This invention generally relates to intravascular medical device systems that navigable through body vessels of a human subject. More particularly, this invention relates to delivery systems and delivery members for delivering and deploying an implantable medical device to a target location of a body vessel and methods of using the same.

### BACKGROUND

The use of catheter delivery systems for positioning and deploying therapeutic devices, such as dilation balloons, stents, and embolic coils, in the vasculature of the human body has become a standard procedure for treating endovascular diseases. It has been found that such devices are particularly useful in treating areas where traditional operational procedures are impossible or pose a great risk to the patient, for example in the treatment of aneurysms in cranial blood vessels. Due to the delicate tissue surrounding cranial blood vessels, e.g. brain tissue, it can be difficult and often risky to perform surgical procedures to treat defects of the cranial blood vessels. Advancements in catheter-based implant delivery systems have provided an alternative treatment in such cases. Some of the advantages of catheter delivery systems are that they provide methods for treating blood vessels by an approach that has been found to reduce the risk of trauma to the surrounding tissue, and they also allow for treatment of blood vessels that in the past would have been considered inoperable.

Typically, these procedures involve inserting a delivery catheter into the vasculature of a patient and guiding it through the vasculature to a predetermined delivery site. A vascular occlusion device, such as an embolic coil, can be attached to an implant engagement/deployment system at a distal end a of a delivery member which pushes the coil through the delivery catheter and out of the distal end of the delivery catheter into the delivery site. Example delivery members and engagement/deployment systems are described in U.S. Patent Application Number 15/850,993, published as U.S. Patent Application Publication No. 2019/0192162 A1 on June 27, 2019, now U.S. Patent No. 10,806,462 issued October 20, 2020 and U.S. Patent Application Number 15/964,857, published as U.S. Patent Application Publication No. 2019/0328398 on October 31, 2019, now U.S. Patent No. 10,806,461 issued October 20, 2020.

Some of the challenges that have been associated with properly executing such treatment procedures include ensuring the delivery member and engagement system remain in a stable position throughout a treatment. For example, in some aneurysm treatment applications, as the aneurysm becomes increasingly packed with embolic material, the delivery member can tend to shift due to increasing pushback from the embolic material being implanted. If the delivery member shifts during treatment, a physician may not be able to accurately control placement of embolic material and may choose to cease packing the aneurysm. In such an example, the aneurysm may not be sufficiently packed, which can lead to recanalization. Further, excessive movement or stretching of the delivery member and/or engagement system thereon can result in premature detachment of the embolic coil.

There is therefore a need for improved methods, devices, and systems to provide an implant delivery member and implant engagement system with increased stability.
The disclosure of EP 3 760 139 A2 provides a delivery member for delivering a deploying an intravascular medical device.
The disclosure of EP 1 188 414 A1 provides a medical device for placement at a predetermined location within a passageway of the human body

### SUMMARY

The presently claimed invention provides a delivery member according to claim 1 and a method of constructing a delivery member according to claim 9. Further developments of the herein claimed invention are described in the dependent claims.

It is an object of the present invention to provide systems, devices, and methods to meet the above-stated needs. Generally, it is an object of the present invention to provide a delivery member for delivering and deploying an implantable medical device having a flexible distal portion.

Stiffness of the distal portion of the delivery member can cause the microcatheter used for delivery of the embolic material to pull back out of the aneurysm as the distal end of the delivery member is advanced through the tortuous distal anatomy. If the microcatheter pulls back while advancing the embolic material, the microcatheter may come out of the aneurysm and the physician may lose control of the embolic coil and not be able to accurately control placement of embolic material and may not be able to complete treatment.

Flexibility can be provided by incorporating a length of wound coil along the distal portion of the delivery member. The wound coil can be protected by a flexible polymer sleeve positioned around the outside of the coil. The wound coil can be inhibited from elongating by a stretch resistant tube affixed to hypotubes on either end of the wound coil.

An example delivery member for delivering an implantable medical device to a target location of a body vessel can include a distal hypotube, a flexible tubular section, a proximal hypotube, a flexible sleeve covering the flexible tubular section, and a stretch resistant member extending across the flexible section. The flexible tubular section can be configured to stretch longitudinally absent the presence of the stretch resistant member. The distal hypotube, flexible tubular section, and proximal hypotube can form a contiguous tubular structure having a lumen therethrough. The flexible sleeve can cover some or all of the flexible tubular section to prevent radial expansion of the flexible tubular section and to promote the ability of the flexible tubular section to slide through vasculature. The stretch resistant member can be affixed to the proximal hypotube and the distal hypotube, thereby extending across the entirety of the flexible tubular section. The stretch resistant member can be positioned outside of the lumen. The flexible sleeve can be affixed to the flexible tubular section and can be fused through openings in the flexible tubular section to the stretch resistant member.

The delivery member can also include an engagement system that can move to engage and deploy the implantable medical device. The engagement system can include a loop wire and a pull wire. The loop wire can extend through an opening in the implantable medical device and the pull wire can be engaged to the loop wire, thereby engaging the engagement system to the implantable medical device. The pull wire can be positioned within the lumen of the delivery member and can be retracted proximally to disengage the loop wire. Once disengaged from the pull wire, the loop wire can be movable to retract from the opening in the implantable medical device, thereby deploying the implantable medical device.

At least a portion of the distal hypotube can be compressed and can elongate upon movement of the engagement system when the engagement system is moved to deploy the implantable medical device.

The flexible tubular section can include a non-radiopaque proximal coil, a non-radiopaque distal coil, and a radiopaque central coil positioned between the non-radiopaque coils.

The flexible tubular section can be made from a wire wound to define a portion of the lumen of the delivery member. The wire from which the flexible tubular is made can have a cross-sectional diameter measuring from about 0.8 mil to about 5 mil.

The flexible sleeve can include a polymer. The flexible sleeve can include additives to increase lubricity of the polymer.

The flexible sleeve can be affixed to the proximal hypotube and the distal hypotube. The flexible sleeve configured thusly can thereby cover the entirety of the coiled section and at least a portion of the proximal hypotube and/or at least a portion of the distal hypotube.

The stretch resistant member can be an extruded tube.

The flexible tubular section and the distal hypotube can have a length measured from the proximal end of the flexible tubular to the distal end of the distal hypotube that measures between about 30 cm and about 50 cm, or more specifically, about 40 cm.

The proximal hypotube can include a spiral cut portion near its distal end.

The delivery member can include a contiguous hypotube which includes the distal hypotube, the proximal hypotube, and the flexible tubular section. The flexible tubular section can have a spiral cut.

An example method for designing or constructing a delivery member such as the example above can include the steps of selecting a first hypotube and a second hypotube, forming a wire coil section between the two hypotubes, extending a stretch resistant member through the lumen of the wire coil section, affixing the stretch resistant member to the first and second hypotubes, selecting a flexible sleeve, covering the flexible tubular section with the flexible sleeve, fusing the flexible sleeve to the stretch resistant tube through openings of the flexible tubular section, and attaching the implantable medical device to the distal end of the first hypotube such that the implantable medical device can be detached from the first hypotube during a treatment.

Another example method for designing or constructing a delivery member such as the example above can include the steps of selecting a first hypotube with a first lumen, selecting a second hypotube with a second lumen, forming a flexible tubular section between the two hypotubes having a third lumen therethrough, extending a stretch resistant member outside of the first, second, and third lumen, affixing a distal portion of the stretch resistant member to the first hypotube and affixing a proximal portion of the stretch resistant member to the second hypotubes, selecting a flexible sleeve, covering the flexible tubular section with the flexible sleeve, fusing the flexible sleeve to the stretch resistant tube through openings of the flexible tubular section, and attaching the implantable medical device to the distal end of the first hypotube such that the implantable medical device can be detached from the first hypotube during a treatment.

The step of forming the wire coil section can include forming a non-radiopaque proximal coil, forming a non-radiopaque distal coil, and forming a radiopaque central coil extending between the non-radiopaque proximal coil and non-radiopaque distal coil. Alternatively, the wire coil section need not include a radiopaque section. The step of forming the wire coil section can additionally or alternatively include selecting a wire having a diameter measuring about 0.8 mil to about 5 mil and winding the wire to form the wire coil section and to define the lumen of the wire coil section.

The step of selecting the flexible sleeve can include selecting a polymer sleeve having additives to increase lubricity of the polymer.

The step of extending the stretch resistant member through the wire coil lumen can include extending a substantially tubular stretch resistant member through the wire coil lumen.

The step of attaching the implantable medical device to the first hypotube can include compressing the first hypotube and attaching the implantable medical device to the distal end of the compressed first hypotube.

The example method for designing or constructing a delivery member can further include positioning a loop wire within the lumen of the first hypotube and positioning a pull wire to extend through lumens of the first hypotube, wire coil section, and the second hypotube. The step of attaching the implantable medical device can additionally or alternatively include extending the loop wire through an opening in the implantable medical device and engaging the pull wire to a portion of the loop wire extended through the opening of the implantable medical device. The step of attaching the implantable medical device can additionally or alternatively include positioning the pull wire to extend proximally from a proximal end of the second hypotube.

Another example method for designing or constructing a delivery member such as the example above can include the steps of spiral cutting a hypotube to form a coiled section in the hypotube such that a distal hypotube section extends distally from the coiled section and a proximal hypotube section extends proximally from the coiled section, extending a stretch resistant tube through a lumen of the coiled section, positioning a flexible sleeve over at least a majority of an outer surface of the coiled section, fusing the flexible sleeve to the stretch resistant tube between windings of the coiled section, and attaching the implantable medical device to the delivery member approximate a distal end of the distal hypotube section.

The example method can further include affixing the stretch resistant tube to the proximal hypotube portion within a lumen of the proximal hypotube portion and affixing the stretch resistant tube to the distal hypotube portion within a lumen of the distal hypotube portion.

The example method can further include extending a pull wire through a lumen of the stretch resistant tube such that the implantable medical device is release upon proximal translation of the pull wire.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of a cross section of a delivery member according to aspects of the present invention;
FIG. 2A is an illustration of a cross section of a flexible sleeve according to aspects of the present invention;
FIG. 2B is an illustration of a cross section of a stretch resistant tube according to aspects of the present invention;
FIG. 2C is an illustration of a cross section of a wire coil affixed to a distal hypotube and a proximal hypotube according to aspects of the present invention;
FIGs. 3A through 3D are illustrations of an engagement system illustrating a sequence for deploying an implant according to aspects of the present invention;
FIG. 4 is a flow diagram illustrating a method for designing or constructing a delivery member according to aspects of the present invention;
FIG. 5 is a flow diagram illustrating a method for using a delivery system including an example delivery member according to aspects of the present invention;
FIGs. 6A and 6B are illustrations of a cross section of a stretch resistant member affixed to a distal hypotube and a proximal hypotube according to aspects of the present invention;
FIGs. 7A and 7B are illustrations of a cross section of a flexible sleeve according to aspects of the present invention; and
FIG. 7C is an illustration of a cross section of a delivery member according to aspects of the present invention.

### DETAILED DESCRIPTION

During an intravascular treatment, for instance, an aneurysm occlusion treatment, lack of flexibility of a distal portion of a treatment device delivery member can cause the delivery member to pull back from the treatment site or otherwise move out of position while an implant or other medical treatment device is being placed in an aneurysm or other treatment site. A delivery member and engagement system having a more flexible distal portion can therefore provide a stable system for delivering medical devices in neurovascular anatomy in addition to other applications facing a similar challenge. Flexible structures, however, can tend deform, extend, or expand when navigating tortuous anatomy. Deformation of the delivery member can inhibit the delivery member's ability to navigate to a treatment site and/or effectively deploy the medical device. Elongation of the delivery member can result in premature deployment of the medical device.

An object of the present invention is to provide a delivery member having a highly flexible distal portion that is stretch resistant and structurally stable throughout delivery and deployment of a medical treatment device. For ease of discussion, medical treatment devices are generally referred to herein as an "implant" although, as will be appreciated and understood by a person of ordinary skill in the art, aspects of the present invention can be applied to deliver and deploy medical treatment devices that are not left implanted.

According to the present invention, in some examples, the highly flexible distal portion of the delivery member can include a coiled wire, an outer sleeve, and an inner stretch resistant member. The coiled wire can be formed of a substantially linear wire that is wound in a coil shape and/or a hypotube that is laser cut in a spiral pattern. If the coiled wire is formed from a laser cut hypotube, the spiral can be absent interference cuts connecting windings in the coil so as to provide a more flexible coil. The outer sleeve can inhibit the coiled wire from deforming radially and/or provide a smooth surface against which vascular walls can slide during delivery of an implant. The stretch resistant member can inhibit elongation of the coiled wire during delivery of the implant. The combination of the coiled wire, outer sleeve, and stretch resistant member can therefore provide a distal portion of a delivery member having greater flexibility and greater stability than at least some known delivery members.

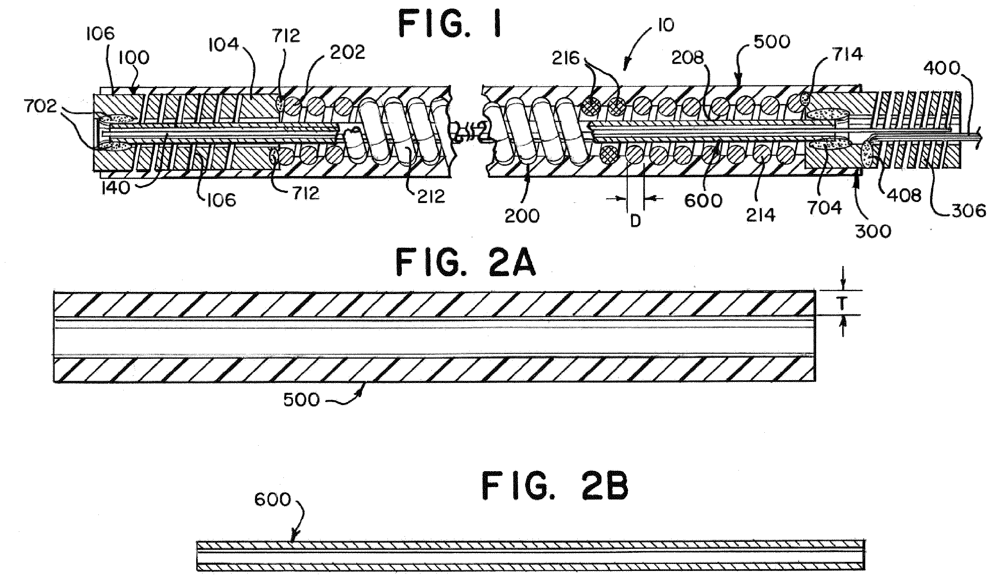

Turning to the figures, as illustrated in FIG. 1, an example delivery member 10 can include a proximal tube 100, a coiled section 200, a distal tube 300, a sleeve 500 surrounding the coiled section, and a stretch resistant member 600 within the lumen of the coiled section 200. The proximal tube 100 can extend a majority of the length of the delivery member 10 with the coiled section 200 and distal tube 300 forming a length sufficient to absorb a majority of push-back that can occur during placement of an implant at a treatment site. In some examples, the length can measure between about 30 cm and about 50 cm, or more specifically, about 40 cm. The proximal tube 100 can have a distal end 104 that is connected to a proximal end 202 of the coiled section 200, and the coiled section 200 can have a distal end 204 that is connected to a proximal end 302 of the distal coil 300.

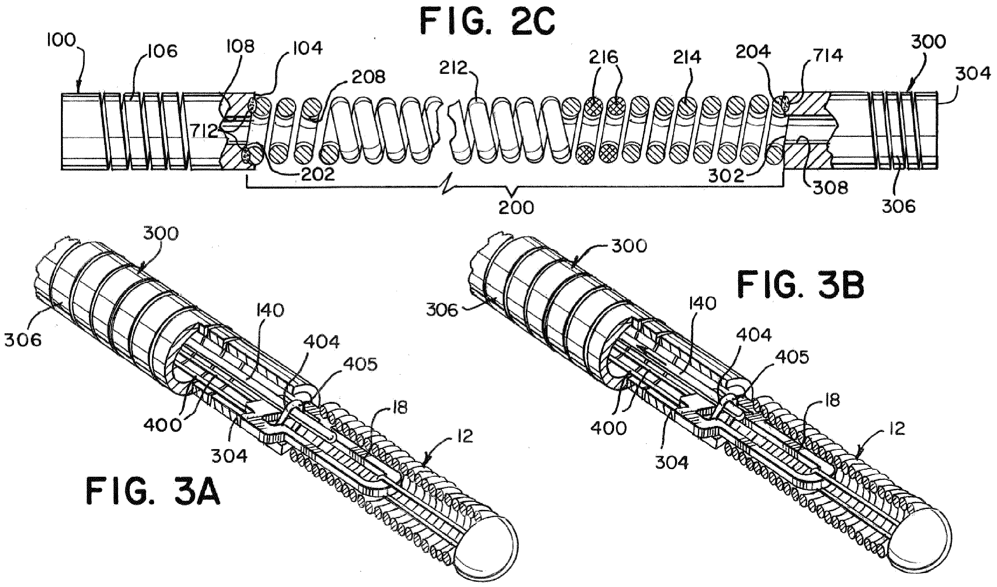

FIG. 2A is a cross sectional view of the sleeve 500. FIG. 2B is a cross sectional view of the stretch resistant member 600. FIG. 2C is a cross sectional view of the assembled proximal tube 100, coiled section 200, and distal tube 300.

The coiled section 200 can be formed separately from the proximal hypotube 100 and/or the distal hypotube 300. The separately formed coiled section 200 can be affixed with welds 712, 714 or other appropriate attachment to the proximal tube 100 and/or the distal tube 300. Alternatively, or additionally, at least a portion of the coiled section can be formed from a spiral laser cut portion of a hypotube. A separately formed coiled section 200 can be made more flexible compared to a spiral cut tube by selecting a wire with a particular cross section with a particular diameter D, or by selecting a wire with material properties to increase flexibility. Conversely, a laser cut portion can be more easily fabricated by cutting a single hypotube to form the proximal tube 100, coiled section 200, and distal hypotube 300, reducing or eliminating welds 712, 714 or other attachments. In either case, the wire of the coil 200 can have a diameter D measuring within a range including about 0.02032 mm (0.8 mils) and 0.127 mm (5 mils).

The coiled section can be formed primarily of a non-radiopaque material such as steel and can include a radiopaque section 216 made of a radiopaque material such as platinum and/or tungsten. The radiopaque section 216 can be positioned between a proximal, non-radiopaque section of the coil 212 and a distal, non-radiopaque section of the coil 214. The radiopaque section 216 can be positioned a predetermined distance from a distal end 304 of the delivery member 10 so that a physician can readily visualize the placement of the distal portion of the delivery member during a treatment procedure. The proximal section 212, radiopaque section 216, and distal section 214 can be concentrically welded.

The coiled section 200 can be surrounded by a flexible sleeve or fused jacket 500, referred generically herein as a "sleeve". The sleeve can inhibit the coil 200 from expanding radially and/or from engaging vascular walls during navigation. The sleeve 500 can include a polymer. The polymer can include additives to increase the lubricity of the sleeve 500 so that the sleeve can easily slide through a body vessel. As illustrated in FIG. 2A, the sleeve 500 can have a wall thickness T measuring within a range including about 0.0127 mm (0.5 mils) and about 0.0508 mm (2 mils). The sleeve 500 can have a wall thickness measuring from about 0.025 mm (0.001 inch) to about 0.076 mm (0.003 inch). The sleeve 500 can further be coated with a hydrophilic coating to further minimize friction during intravascular navigation. The sleeve 500 can be fused or glued to the coil 200, the proximal hypotube 100, and/or the distal hypotube 300. The flexible sleeve 500 can be fused through openings of the coil 200 to the stretch resistant member 600.

The stretch resistant member 600 can be positioned to inhibit elongation of the coil 200 during intravascular navigation. The stretch resistant member 600 can include a tube sized to fit within the lumen 208 of the coil 200. The stretch resistant tube 600 can also be sized to extend through the entirety of the length of the coil 200, extend with a lumen 108 of the proximal tube 100 and within the lumen 308 of the distal coil 300. The stretch resistant member 600 can be attached to the proximal tube 100 and the distal tube 300 at adhesive joints 702, 704 or other appropriate attachment. The stretch resistant member 600 can remain unattached to the coiled section 200 such that the stretch resistant member 600 and coiled section 200 are able to move independently from each other to some extent.

The delivery member 10 can include a mechanical engagement system for engaging a medical treatment device during delivery to a treatment site that can be actuated mechanically to deploy the treatment device. Mechanically actuated engagement systems often include one or more inner elongated members or pull wires extending through the delivery member that can be manipulated at the proximal end by a physician to deploy a medical treatment device. Such a wire or inner elongated member is referred to herein generically as a "pull wire".

FIGs. 3A through 3D illustrate the delivery member 10 including a mechanical engagement system including a pull wire 140 and a loop wire 400 that can be positioned to secure an implant or other medical treatment device to the delivery member 10 and can be moved to release the medical treatment device from the delivery member 10. The loop wire 400 can be affixed to the distal tube 300 with a weld 408 or other or other suitable attachment. The stretch resistant member 600 can be sized to allow a pull wire 140 to pass through the lumens 108, 208, 308 of the proximal tube 100, coiled section 200, and distal tube 300. For instance, the stretch resistant member 600 can be tubular, having a lumen therethrough, and the pull wire 140 can extend through the lumen of the tubular stretch resistant member 600. During manufacture of the stretch resistant member 600, the stretch resistant member 600 can be extruded over the pull wire 140.

The combination of the coil 200, sleeve 500, and stretch resistant member 600 can provide a highly flexible distal portion of a delivery member 10 suitable for navigating tortuous anatomy, including neurovascular blood vessels. The stretch resistant member 600 can support the coil 200 to prevent the coil 200 from significantly extending during navigation of a blood vessel, thereby reducing tension on a pull wire 140 extending therethrough and reducing the likelihood of premature deployment of an attached medical treatment device.

The proximal tube 100 can include a flexible section 106 having material removed to increase flexibility of the flexible section 106. The flexible section 106 can be cut in a spiral pattern. The spiral pattern of the flexible section 106 can lack interference cuts connecting windings within the spiral. The stretch resistant member 600 can extend through the flexible section 106 and be attached to the proximal tube 100 in the proximal direction from the flexible section 106. The stretch resistant member 600 can thereby inhibit elongation of the flexible section 106 of the proximal tube 100 and coiled section 200. The sleeve 500 can cover at least a portion of the flexible section 106 to inhibit deformation of the flexible section and/or reduce friction with vasculature and the flexible section 106 during intravascular navigation. In some examples, the sleeve 500 can cover about 10 cm of the proximal tube 100 approximate and/or including the distal end 104 of the proximal tube 100.

The distal tube 300 can include a compressible portion 306. The compressible portion 306 can be axially adjustable between an elongated condition and a compressed condition. The compressed portion 306 can be formed from a spiral-cut portion of the tube 300, formed by a laser cutting operation. Additionally, or alternatively, the compressible portion can be formed of a wound wire, spiral ribbon, or other arrangement allowing axial adjustment according to the present invention. Preferably, the compressible portion 306 is in the elongated condition at rest and automatically or resiliently returns to the elongated condition from a compressed condition, unless otherwise constrained.

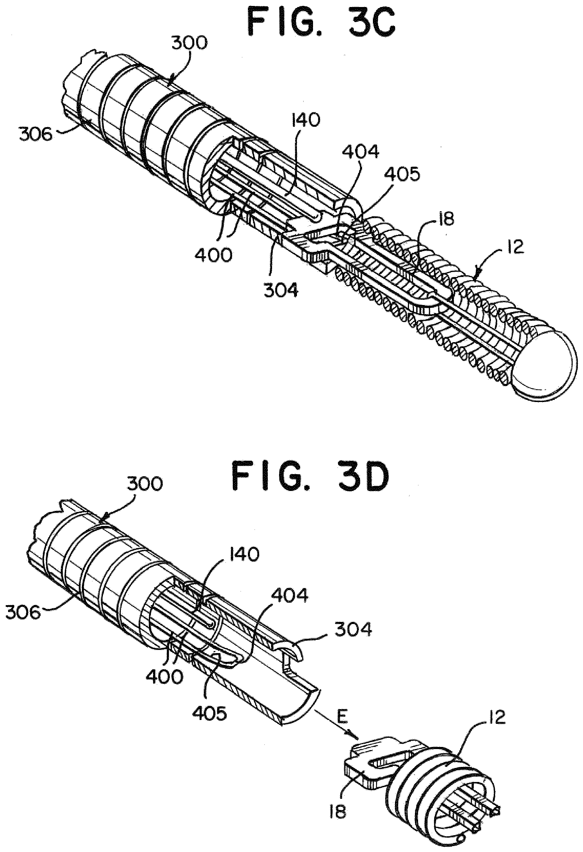

FIGs. 3A-3D, illustrate the detachment of the medical device 12 using a mechanical engagement/deployment system. FIG. 3A illustrates the engagement system 140, 400 locked into the locking portion 18 of the medical device 12. The compressible portion 306 of the distal tube 300 can be compressed and the loop wire 400 opening 405 at a distal end 404 of the loop wire 400 can be placed through the locking portion 18. When the pull wire 140 is put through the opening 405 the medical device 12 is now secure. FIG. 3B illustrates the pull wire 140 being drawn proximally to begin the release sequence for the medical device 12. FIG. 3C illustrates the instant the pull wire 140 exits the opening 405 and is pulled free of the loop wire 400. The distal end 404 of the loop wire 400 falls away and exits the locking portion 18. As can be seen, there is now nothing holding the medical device 12 to the detachment system 10. FIG. 3D illustrates the end of the release sequence. Here, the compressible portion 306 has extended/returned to its original shape and "sprung" forward. An elastic force E is imparted by the distal end 304 of the distal tube 300 to the medical device 12 to "push" it away to ensure a clean separation and delivery of the medical device 12.

Illustrations in the above-described figures depict generally hollow or tubular structures 100, 200, 300, 500, 600 according to the present invention. When used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

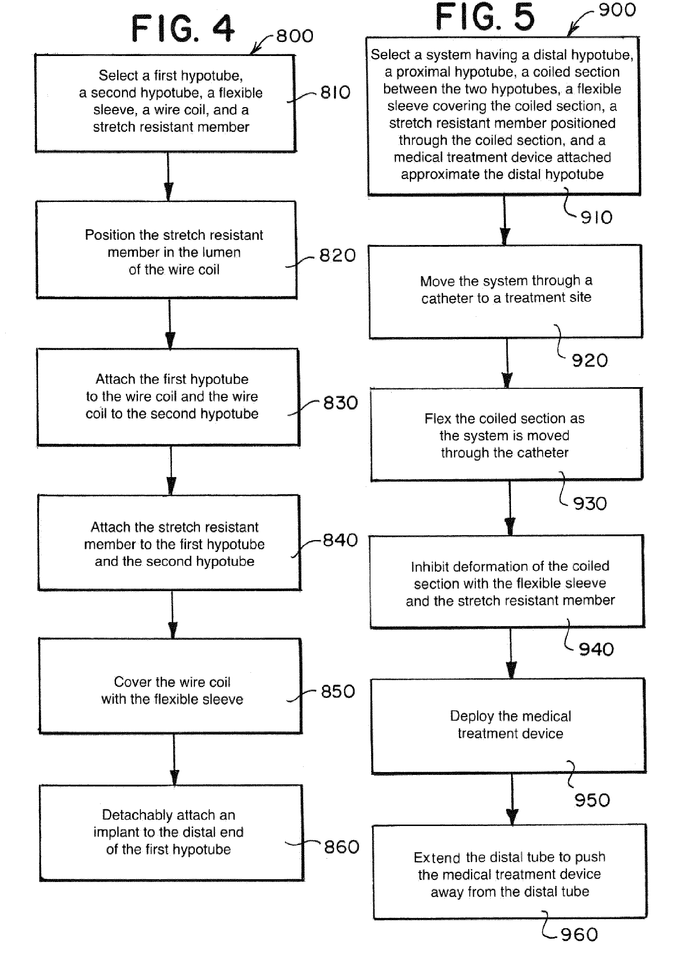

FIG. 4 is a flow diagram including method steps for constructing or designing a delivery member such as the example delivery members described herein. Referring to the method 800 outlined in FIG. 4, in step 810, a first hypotube, a second hypotube, a flexible sleeve, a wire coil, and a stretch resistant member can be selected. The first hypotube can be a proximal hypotube 100 as described herein or as would otherwise be known to a person of ordinary skill in the art. The second hypotube can be a distal hypotube 300 as described herein or as would otherwise be known to a person of ordinary skill in the art. The flexible sleeve can be a sleeve or fused jacket 500 as described herein or as otherwise known to a person of ordinary skill in the art. The wire coil can include the support coil, coiled section 200 as described herein or as otherwise known to a person of ordinary skill in the art. The stretch resistant member can be a stretch resistant member 600 as described herein or as otherwise known to a person of ordinary skill in the art.

In step 820, the stretch resistant member can be positioned in the lumen of the wire coil. In step 820, the stretch resistant member that is positioned can be substantially tubular. In step 830, the first hypotube, wire coil, and second hypotube can be attached to each other. In step 840, the stretch resistant member is attached to the first hypotube and the second hypotube. The first hypotube, wire coil, and second hypotube can be attached as illustrated and described herein or by other means as would be understood by a person of ordinary skill in the art. Steps 820, 830, and 840 need not be performed in that order and can be performed simultaneously. For instance, the stretch resistant member can be attached to one of the first and second hypotubes as indicated in step 840, then the hypotube to which the stretch resistant member is attached can be attached to the wire coil as indicated in step 830, then the stretch resistant member can be positioned through the wire coil as indicated in step 820, then the other of the hypotubes can be attached to the wire coil as indicated in step 830, then the stretch resistant member can be attached to that other hypotube as indicated in step 840.

In step 850, the wire coil can be covered with the flexible sleeve. The flexible sleeve can cover some or all of the outer surface of the wire coil. Step 850 can also include the step of fusing the flexible sleeve to the wire coil and/or otherwise affixing the flexible sleeve to the delivery member. Step 850 can also include the step of fusing the flexible sleeve to the stretch resistant member. If the second hypotube has a flexible section, in step 850, the flexible sleeve can also be positioned to cover at least a portion of the flexible section.

In step 860, an implant can be detachably attached to the distal end of the first hypotube. In step 860, the implant can be attached by positioning a loop wire within the first hypotube, positioning a pull wire to extend through the first hypotube, coiled wire, and second hypotube, and securing the implant with the loop wire and the pull wire. The pull wire can be extended from the proximal end of the second hypotube. If the first hypotube has a compressible portion, in step 860, the compressible portion can be compressed, and the implant can be attached to delivery member while the compressible portion is compressed.

FIG. 5 is a flow diagram including method steps for administering an intravascular treatment using a system including a delivery member such as the example delivery members described herein. Referring to the method 900 outlined in FIG. 5, in step 910 a system having a distal hypotube, proximal hypotube, coiled section co-axially positioned in between the hypotubes, a flexible sleeve covering the coiled section, a stretch resistant member positioned within the coiled section, and a medical treatment device attached to or near the distal hypotube can be selected. The system can be suitable for intravascular treatments such as described and illustrated herein or as otherwise known to a person of ordinary skill in the art.

In step 920, the system can be moved through a catheter to a treatment site such as the site of an aneurysm or other abnormality in a blood vessel. In step 930, the system can be flexed as it is moved through the catheter. In step 940, the coiled section of the system can be prevented from deforming by the flexible sleeve and the stretch resistant member; the flexible sleeve can inhibit the coiled section from deforming radially while the stretch resistant member can inhibit the coil from extending longitudinally.

In step 950, the medical treatment device can be deployed. In the case that the medical treatment device is an implant, in step 950 the implant can be detached. In step 960, the distal tube can extend to push the medical treatment device away from the distal tube. In the case that the medical treatment device is an implant detached in step 950, in step 960, the detached implant can be ejected away from the distal tube in response to the expansion of the distal tube.

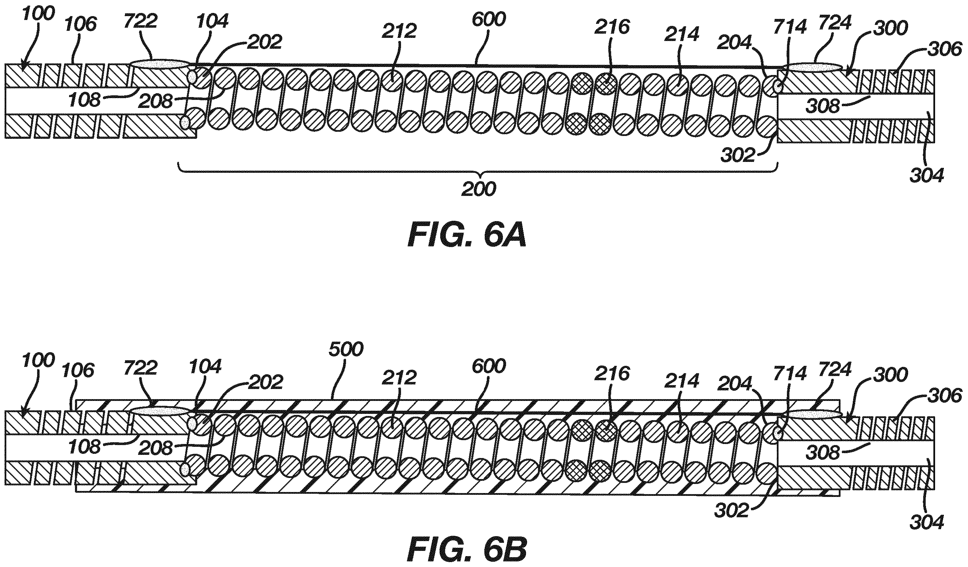

FIG. 6A is an illustration of a cross section of a stretch resistant member 600 affixed to a distal hypotube 300 and a proximal hypotube 100 according to aspects of the present invention. The stretch resistant member 600 can be positioned to inhibit elongation of the flexible tubular section 200 during intravascular navigation. The stretch resistant member 600 can be positioned outside the lumen 208 of the flexible tubular section 200 and outside the lumen 108, 308 of either or both of the proximal hypotube 100 and distal hypotube 100. The stretch resistant member 600 can also be sized to extend along the entirety of the length of the flexible tubular section 200, extend to the proximal hypotube 100 and extend to the distal hypotube 300. The stretch resistant member 600 can be attached to the proximal tube 100 and the distal tube 300 at adhesive joints 722, 724 or other appropriate attachment. The stretch resistant member 600 can remain unattached to the flexible tubular section 200 such that the stretch resistant member 600 and flexible tubular section 200 are able to move independently from each other, to some extent.

FIG. 6B is an illustration of a cross section of a flexible sleeve 500 positioned over the stretch resistant member 600 affixed to the distal hypotube 300 and the proximal hypotube 100 according to aspects of the present invention. The flexible sleeve 500 can have a wall thickness T measuring within a range including about 0.025 mm (0.001 inch) to about 0.076 mm (0.003 inch). The flexible sleeve 500 can further be coated with a hydrophilic coating to further minimize friction during intravascular navigation. The flexible sleeve 500 can be fused or glued to the flexible tubular section 200, the proximal hypotube 100, and/or the distal hypotube 300, such that the flexible sleeve 500 prevents the flexible tubular section 200 from stretching while the delivery member 10 is manipulated in the vasculature, while also preserving the flexibility of the distal hypotube 300. The flexible sleeve 500 can be fused through openings of the flexible tubular section 200.

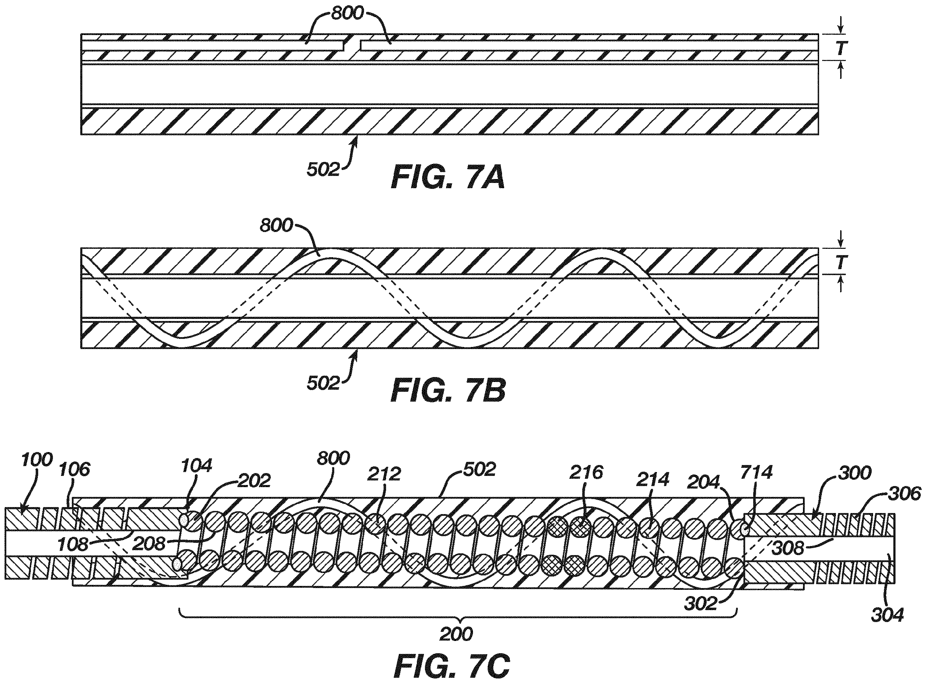

As illustrated in FIGs. 7A and 7B, the sleeve 502 can have one or more stretch resistant fibers 800 positioned within a wall of the sleeve 502. The stretch resistant fibers 800 can include polymeric fibers and/or metallic fibers. The stretch resistant fibers 800 can be oriented within the wall of the sleeve 502 in a linear orientation, as shown in FIG. 7A, or in one or more helical orientations, as shown in FIG. 7B. The stretch resistant fibers 800 can be incorporated into the wall of the sleeve 502 during the extrusion process of the fibered sleeve 502. The fibered sleeve 502 can have a wall thickness T measuring within a range including about 0.025 mm (0.001 inch) to about 0.076 mm (0.003 inch). The fibered sleeve 502 can further be coated with a hydrophilic coating to further minimize friction during intravascular navigation.

FIG. 7C is an illustration of a cross section of a delivery member 10 according to aspects of the present invention. The fibered sleeve 502 can be fused or glued to the flexible tubular section 200, the proximal hypotube 100, and/or the distal hypotube 300, such that the fibered sleeve 502 prevents the flexible tubular section 200 from stretching while the delivery member 10 is manipulated in the vasculature, while also preserving the flexibility of the distal hypotube 300. The fibered sleeve 502 can also be fused through openings of the flexible tubular section 200.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ± 20% of the recited value, e.g. "about 90%" may refer to the range of values from 71% to 99%.

## Claims

1. A delivery member for delivering an implantable medical device to a target location of a body vessel, the delivery member comprising:
a distal hypotube (300) comprising a distal end (304) shaped to receive the implantable medical device;
a flexible tubular section (200) affixed to a proximal end (302) of the distal hypotube, the flexible tubular section comprising openings therethrough;
a proximal hypotube (100) affixed to a proximal end (202) of the flexible tubular section;
a lumen (208) extending through the distal hypotube, the flexible tubular section, and the proximal hypotube;
**characterised by**:
a stretch resistant member (600) positioned outside of the lumen, affixed to the proximal hypotube, affixed to the distal hypotube, and extending along at least a portion of an outer surface of the flexible tubular section; and
a flexible sleeve (500) covering at least a majority of the outer surface of the flexible tubular section and stretch resistant member.

2. The delivery member of claim 1, further comprising:
an engagement system (140, 400) movable to engage and deploy an implantable medical device engaged at the distal end of the distal hypotube, the engagement system comprising:
a loop wire (400) extended through an opening in the implantable medical device thereby engaging the engagement system to the implantable medical device and movable to retract from the opening in the implantable medical device to deploy the implantable medical device, and
a pull wire (140) extended through the lumen, engaged to the loop wire thereby engaging the engagement system to the implantable medical device, and movable to retract proximally to disengage the loop wire to deploy the implantable medical device.

3. The delivery member of claim 2,
wherein the distal hypotube comprises a compressible portion (306) movable from a compressed condition to an elongated condition, and
wherein the engagement system maintains the compressible portion in the compressed condition when engaged to the implantable medical device.

4. The delivery member of claim 1, wherein the flexible tubular section comprises:
a non-radiopaque proximal coil (212) extending from the proximal end of the flexible tubular section;
a non-radiopaque distal coil (214) extending from the distal end of the flexible tubular section; and
a radiopaque central coil (216) extending between the non-radiopaque proximal coil and the non-radiopaque distal coil.

5. The delivery member of claim 1, where in the flexible tubular section comprises:
a wire wound to form the flexible tubular section and defining a portion of the lumen, the wire comprising a diameter measuring from about 0.02 mm (0.0008 inch) to about 0.1 mm (0.005 inch).

6. The delivery member of claim 1,
wherein the flexible sleeve comprises a polymer, and
wherein the flexible sleeve comprises additives effective to increase lubricity of the polymer.

7. The delivery member of claim 1, wherein the flexible sleeve is affixed to the proximal hypotube and the distal hypotube.

8. The delivery member of claim 1, wherein the delivery member comprises a length measurable from the proximal end of the flexible tubular section to the distal end of the distal hypotube, and wherein the length measures about 40 cm.

9. A method of constructing a delivery member for delivering an implantable medical device, the method comprising:
selecting a first hypotube (300) comprising a first lumen therethrough;
selecting a second hypotube (100) comprising a second lumen therethrough;
forming a flexible tubular section (200) extending from a distal end (104) of the second hypotube to a proximal end (302) of the first hypotube such that the flexible tubular section defines a third lumen therethrough;
affixing a distal portion of a stretch resistant member (600) to the first hypotube and affixing a proximal portion of the stretch resistant member to the second hypotube,
characterised wherein an intermediate portion of the stretch resistant member is positioned outside of the first, second, and third lumen;
selecting a flexible sleeve (500);
covering at least a majority of an outer surface of the flexible tubular section with the flexible sleeve; and
detachably attaching the implantable medical device to the delivery member approximate a distal end of the first hypotube.

10. The method of claim 9, wherein the step of selecting the flexible sleeve further comprises:
selecting the flexible sleeve comprising a polymer and further comprising at least one of:
an additive effective to increase lubricity of the polymer,
a stretch resistant fiber comprising a polymer, and
a stretch resistant fiber comprising a metal.

11. The method of claim 9, wherein the step of detachably attaching the implantable medical device to the delivery member approximate a distal end of the first hypotube further comprises:
compressing the first hypotube; and
detachably attaching the implantable medical device to the delivery member approximate the distal end of the compressed first hypotube.

## Patentansprüche

1. Abgabeelement zum Abgeben einer implantierbaren medizinischen Vorrichtung an eine Zielstelle eines Körpergefäßes, das Abgabeelement umfassend:
ein distales Hyporohr (300), umfassend ein distales Ende (304), das geformt ist, um die implantierbare medizinische Vorrichtung aufzunehmen;
einen flexiblen rohrförmigen Abschnitt (200), der an einem proximalen Ende (302) des distalen Hyporohrs befestigt ist, der flexible rohrförmige Abschnitt umfassend Öffnungen dahindurch;
ein proximales Hyporohr (100), das an einem proximalen Ende (202) des flexiblen rohrförmigen Abschnitts befestigt ist;
ein Lumen (208), das sich durch das distale Hyporohr, den flexiblen rohrförmigen Abschnitt und das proximale Hyporohr erstreckt;
**gekennzeichnet durch:**
ein dehnungsbeständiges Element (600), das außerhalb des Lumens positioniert ist, an dem proximalen Hyporohr befestigt ist, an dem distalen Hyporohr befestigt ist und sich entlang mindestens eines Anteils einer Außenoberfläche des flexiblen rohrförmigen Abschnitts erstreckt; und
eine flexible Hülse (500), die mindestens einen Großteil der Außenoberfläche des flexiblen rohrförmigen Abschnitts und des dehnungsbeständigen Elements bedeckt.

2. Abgabeelement nach Anspruch 1, ferner umfassend:
ein Eingriffssystem (140, 400), das beweglich ist, um eine implantierbare medizinische Vorrichtung, die an dem distalen Ende des distalen Hyporohrs in Eingriff steht, in Eingriff zu nehmen und einzusetzen, das Eingriffssystem umfassend:
einen Schleifendraht (400), der sich durch eine Öffnung in der implantierbaren medizinischen Vorrichtung erstreckt, wodurch das Eingriffssystem die implantierbare medizinische Vorrichtung in Eingriff nimmt, und der beweglich ist, um aus der Öffnung in der implantierbaren medizinischen Vorrichtung zurückgezogen zu werden, um die implantierbare medizinische Vorrichtung einzusetzen, und
einen Zugdraht (140), der sich durch das Lumen erstreckt, der mit dem Schleifendraht in Eingriff steht und dadurch das Eingriffssystem mit der implantierbaren medizinischen Vorrichtung in Eingriff bringt, und der beweglich ist, um proximal zurückgezogen zu werden, um den Schleifendraht zu lösen, um die implantierbare medizinische Vorrichtung einzusetzen.

3. Abgabeelement nach Anspruch 2,
wobei das distale Hyporohr einen komprimierbaren Anteil (306) umfasst, der von einem komprimierten Zustand in einen verlängerten Zustand beweglich ist, und
wobei das Eingriffssystem den komprimierbaren Anteil in dem komprimierten Zustand hält, wenn er mit der implantierbaren medizinischen Vorrichtung in Eingriff steht.

4. Abgabeelement nach Anspruch 1, wobei der flexible rohrförmige Abschnitt umfasst:
eine nicht röntgendichte proximale Spule (212), die sich von dem proximalen Ende des flexiblen rohrförmigen Abschnitts erstreckt;
eine nicht röntgendichte distale Spule (214), die sich von dem distalen Ende des flexiblen rohrförmigen Abschnitts erstreckt; und
eine röntgendichte zentrale Spule (216), die sich zwischen der nicht röntgendichten proximalen Spule und der nicht röntgendichten distalen Spule erstreckt.

5. Abgabeelement nach Anspruch 1, wobei der flexible rohrförmige Abschnitt umfasst:
einen Draht, der gewickelt ist, um den flexiblen rohrförmigen Abschnitt auszubilden und einen Anteil des Lumens zu definieren, der Draht umfassend einen Durchmesser von etwa 0,02 mm (0,0008 Zoll) bis etwa 0,1 mm (0,005 Zoll).

6. Abgabeelement nach Anspruch 1,
wobei die flexible Hülse ein Polymer umfasst, und
wobei die flexible Hülse Additive umfasst, die wirksam sind, um eine Gleitfähigkeit des Polymers zu erhöhen.

7. Abgabeelement nach Anspruch 1, wobei die flexible Hülse an dem proximalen Hyporohr und dem distalen Hyporohr befestigt ist.

8. Abgabeelement nach Anspruch 1, wobei das Abgabeelement eine Länge umfasst, die von dem proximalen Ende des flexiblen rohrförmigen Abschnitts zu dem distalen Ende des distalen Hyporohrs messbar ist, und wobei die Länge etwa 40 cm misst.

9. Verfahren zum Konstruieren eines Abgabeelements zum Abgeben einer implantierbaren medizinischen Vorrichtung, das Verfahren umfassend:
Auswählen eines ersten Hyporohrs (300), umfassend ein erstes Lumen dahindurch;
Auswählen eines zweiten Hyporohrs (100), umfassend ein zweites Lumen dahindurch;
Ausbilden eines flexiblen rohrförmigen Abschnitts (200), der sich von einem distalen Ende (104) des zweiten Hyporohrs zu einem proximalen Ende (302) des ersten Hyporohrs derart erstreckt, dass der flexible rohrförmige Abschnitt ein drittes Lumen dahindurch definiert;
Befestigen eines distalen Anteils eines dehnungsbeständigen Elements (600) an dem ersten Hyporohr und Befestigen eines proximalen Anteils des dehnungsbeständigen Elements an dem zweiten Hyporohr,
**gekennzeichnet** wobei ein Zwischenanteil des dehnungsbeständigen Elements außerhalb des ersten, zweiten und dritten Lumens positioniert ist;
Auswählen einer flexiblen Hülse (500);
Bedecken von mindestens einem Großteil der Außenoberfläche des flexiblen rohrförmigen Abschnitts mit der flexiblen Hülse; und
lösbares Befestigen der implantierbaren medizinischen Vorrichtung an dem Abgabeelement in der Nähe eines distalen Endes des ersten Hyporohrs.

10. Verfahren nach Anspruch 9, wobei der Schritt des Auswählens der flexiblen Hülse ferner umfasst:
Auswählen der flexiblen Hülse, umfassend ein Polymer, und ferner umfassend mindestens eines von:
einem Additiv, das wirksam ist, um die Gleitfähigkeit des Polymers zu erhöhen,
einer dehnungsbeständigen Faser, umfassend ein Polymer, und
einer dehnungsbeständigen Faser, umfassend ein Metall.

11. Verfahren nach Anspruch 9, wobei der Schritt des lösbaren Befestigens der implantierbaren medizinischen Vorrichtung an dem Abgabeelement in der Nähe eines distalen Endes des ersten Hyporohrs ferner umfasst:
Komprimieren des ersten Hyporohrs; und
lösbares Befestigen der implantierbaren medizinischen Vorrichtung an dem Abgabeelement in der Nähe des distalen Endes des komprimierten ersten Hyporohrs.

## Revendications

1. Élément de libération permettant de libérer un dispositif médical implantable à un emplacement cible d'un vaisseau corporel, l'élément de libération comprenant :
un hypotube distal (300) comprenant une extrémité distale (304) profilée pour recevoir le dispositif médical implantable ;
une section tubulaire souple (200) fixée à une extrémité proximale (302) de l'hypotube distal, la section tubulaire souple comprenant des ouvertures à travers celle-ci ;
un hypotube proximal (100) fixé à une extrémité proximale (202) de la section tubulaire souple ;
une lumière (208) s'étendant à travers l'hypotube distal, la section tubulaire souple, et l'hypotube proximal ;
**caractérisé par** :
un élément résistant à l'étirement (600) positionné à l'extérieur de la lumière, fixé à l'hypotube proximal, fixé à l'hypotube distal, et s'étendant le long d'au moins une partie d'une surface externe de la section tubulaire souple ; et
un manchon souple (500) couvrant au moins une majorité de la surface externe de la section tubulaire souple et de l'élément résistant à l'étirement.

2. Élément de libération selon la revendication 1, comprenant en outre :
un système de mise en prise (140, 400) mobile pour venir en prise et déployer un dispositif médical implantable en prise au niveau de l'extrémité distale de l'hypotube distal, le système de mise en prise comprenant :
un fil de boucle (400) étendu à travers une ouverture dans le dispositif médical implantable mettant en prise de ce fait le système de mise en prise avec le dispositif médical implantable et mobile pour se rétracter de l'ouverture dans le dispositif médical implantable pour déployer le dispositif médical implantable, et
un fil de traction (140) étendu à travers la lumière, en prise avec le fil de boucle mettant en prise de ce fait le système de mise en prise avec le dispositif médical implantable, et mobile pour se rétracter de manière proximale pour désengager le fil de boucle pour déployer le dispositif médical implantable.

3. Élément de libération selon la revendication 2,
dans lequel l'hypotube distal comprend une partie compressible (306) mobile d'un état compressé à un état allongé, et
dans lequel le système de mise en prise maintient la partie compressible dans l'état compressé lorsqu'en prise avec le dispositif médical implantable.

4. Élément de libération selon la revendication 1, dans lequel la section tubulaire souple comprend :
une bobine proximale non radio-opaque (212) s'étendant de l'extrémité proximale de la section tubulaire souple ;
une bobine distale non radio-opaque (214) s'étendant de l'extrémité distale de la section tubulaire souple ; et
une bobine centrale radio-opaque (216) s'étendant entre la bobine proximale non radio-opaque et la bobine distale non radio-opaque.

5. Élément de libération selon la revendication 1, dans lequel la section tubulaire souple comprend :
un fil enroulé pour former la section tubulaire souple et définissant une partie de la lumière, le fil comprenant un diamètre mesurant d'environ 0,02 mm (0,0008 pouce) à environ 0,1 mm (0,005 pouce).

6. Élément de libération selon la revendication 1,
dans lequel le manchon souple comprend un polymère, et
dans lequel le manchon souple comprend des additifs efficaces pour augmenter le pouvoir lubrifiant du polymère.

7. Élément de libération selon la revendication 1, dans lequel le manchon souple est fixé à l'hypotube proximal et à l'hypotube distal.

8. Élément de libération selon la revendication 1, dans lequel l'élément de libération comprend une longueur mesurable de l'extrémité proximale de la section tubulaire souple à l'extrémité distale de l'hypotube distal, et dans lequel la longueur mesure environ 40 cm.

9. Procédé de construction d'un élément de libération pour la libération d'un dispositif médical implantable, le procédé comprenant :
la sélection d'un premier hypotube (300) comprenant une première lumière à travers celui-ci ;
la sélection d'un second hypotube (100) comprenant une deuxième lumière à travers celui-ci ;
la formation d'une section tubulaire souple (200) s'étendant d'une extrémité distale (104) du second hypotube à une extrémité proximale (302) du premier hypotube de telle sorte que la section tubulaire souple définit une troisième lumière à travers celui-ci ;
la fixation d'une partie distale d'un élément résistant à l'étirement (600) au premier hypotube et la fixation d'une partie proximale de l'élément résistant à l'étirement au second hypotube,
**caractérisé** dans lequel une partie intermédiaire de l'élément résistant à l'étirement est positionnée à l'extérieur de la première, de la deuxième, et de la troisième lumière ;
la sélection d'un manchon souple (500) ;
le recouvrement d'au moins une majorité de la surface externe de la section tubulaire souple avec le manchon souple ; et
l'attachement de manière amovible du dispositif médical implantable à l'élément de libération à proximité d'une extrémité distale du premier hypotube.

10. Procédé selon la revendication 9, dans lequel l'étape de sélection du manchon souple comprend en outre :
la sélection du manchon souple comprenant un polymère et comprenant en outre au moins l'un parmi :
un additif efficace pour augmenter le pouvoir lubrifiant du polymère,
une fibre résistante à l'étirement comprenant un polymère, et
une fibre résistante à l'étirement comprenant un métal.

11. Procédé selon la revendication 9, dans lequel l'étape d'attachement de manière amovible du dispositif médical implantable à l'élément de libération à proximité d'une extrémité distale du premier hypotube comprend en outre :
la compression du premier hypotube ; et
l'attachement de manière amovible du dispositif médical implantable à l'élément de libération à proximité de l'extrémité distale du premier hypotube compressé.
